# FASCICULE DE BREVET EUROPEEN

(11) **EP 0 711 359 B1**
(45) Date de publication et mention de la délivrance du brevet: **28.05.1997**
(21) Numéro de dépôt: 94923760.6
(22) Date de dépôt: 28.07.1994
(51) Int. Cl.: C12Q 1/04

(54) **PROCEDE D'IDENTIFICATION DE MICROORGANISMES AVEC UN MILIEU SUPPLEMENTE EN HYDRATE DE CARBONE**
VERFAHREN ZUR IDENTIFIZIERUNG VON MIKROORGANISMEN MITTELS EINES MIT KOHLEHYDRATEN ANGEREICHERTEN MEDIUMS
MICROORGANISM IDENTIFICATION METHOD USING A MEDIUM CONTAINING ADDED CARBOHYDRATE

(30) Priorité: 28.07.1993 FR 9309293
(43) Date de publication de la demande: 15.05.1996
(73) Titulaire: Rambach, Alain, F-75006 Paris (FR)
(72) Inventeur: Rambach, Alain, F-75006 Paris (FR)
(74) Mandataire: Warcoin, Jacques
(86) Numéro de dépôt international: FR9400957
(87) Numéro de publication internationale: WO9504156

(56) Documents cités:
- EP-A- 0 065 137
- EP-A- 0 395 532
- WO-A-90/12888
- WO-A-92/12259
- DE-A- 2 717 978
- FR-A- 2 684 110
- US-A- 5 210 022

## Description

La présente invention concerne un procédé de mise en évidence de la présence ou de l'absence d'une souche de microorganisme particulière dans un milieu de culture.

La détection de microorganisme est très importante notamment dans l'industrie alimentaire, au niveau du contrôle des eaux ou en médecine, sachant que ces microorganismes peuvent non seulement se révéler être des agents pathogènes, mais également consister en des agents mettant en évidence certains types de contaminations.

Différents procédés permettent de mettre en évidence la présence de microorganismes, dans un milieu quelconque, consistant à prélever un échantillon du milieu considéré, puis à favoriser le développement des microorganismes présents par culture sur ou dans un milieu approprié.

De manière à simplifier la mise en évidence des microorganismes présents, il a été proposé d'utiliser dans le milieu de détection des composés colorés dont la présence est caractéristique d'un microorganisme donné.

La coloration est souvent révélatrice d'une activité enzymatique liée au microorganisme considéré, et le résultat de cette activité peut résulter en une modification du pH du milieu, mise en évidence par un indicateur coloré (EP-A-0 395 532), ou encore en la libération d'un composé chromophore ou fluorophore (FR-A-2 684 110).

Les chromophores ou fluorophores sont des composés généralement obtenus par hydrolyse enzymatique de composés chromogènes ou fluorogènes correspondants, présents dans le milieu de culture.

Les fluorophores émettent un rayonnement caractéristique par fluorescence.

Les composés chromophores sont caractérisés par une couleur d'une longueur d'onde dominante.

Parmi les composés chromophores connus, on notera notamment les dérivés de l'indoxyle, de l'hydroquinoline ou encore les dérivés naphtoïques, ou les dérivés du naphtyle et du phényle.

De manière à différencier deux genres de microorganismes différents dans un milieu de culture, il a même été proposé d'introduire deux agents chromogènes, chacun libérant un composé chromophore d'une couleur caractéristique de la présence d'un microorganisme particulier (US-A-5 210 022).

Bien que l'ensemble de ces milieux soit performant pour la détection de microorganismes d'un genre spécifique, comme par exemple Salmonella, Candida ou E.coli, et leur distinction parmi d'autres espèces, ils ne permettent toutefois pas la détection d'un grand nombre de microorganismes de genres différents sur un même milieu de culture, ou encore parmi les microorganismes d'un même genre la différenciation des espèces pathogènes des autres.

Une telle distinction parait d'autant plus importante pour certaines espèces de levures comme Candida albicans qui est responsable de plus de 50% des pathologies liées aux levures.

Or, il a été trouvé d'une manière inattendue que l'on pouvait remédier à ces inconvénients en introduisant dans le milieu de culture au moins un chromogène substrat d'une enzyme de la souche et au moins un composé choisi parmi un hydrate de carbone à concentration élevée, afin d'obtenir une couleur dérivée différente de la couleur de base du chromophore.

Par couleur dérivée, on entend toute couleur dont la longueur d'onde dominante diffère de la longueur d'onde dominante des chromophores libérés par les chromogènes présents dans le milieu de culture pris isolément dans un milieu standard.

Par milieu standard, on entend tout milieu usuel d'identification dans lequel l'hydrate de carbone a une simple fonction de source de carbone, à des concentrations très faibles, voire nulle, où il est considéré que de telles concentrations permettent d'éviter tout effet inducteur qui modifierait d'une manière incontrôlée le comportement des microorganismes et induirait des erreurs dans l'identification des microorganismes considérés.

La longueur d'onde dominante du chromophore pourra être calculée en référence à la lumière du jour, telle que définie par la CIE (Commission Internationale de l'Energie) comme illuminant D₆₅, à partir de toute méthode standard de mesure de couleur d'objet, en particulier avec un spectrocolorimètre.

La présente invention concerne donc un procédé de mise en évidence de la présence ou de l'absence d'une souche de microorganisme particulière dans un milieu, caractérisé en ce qu'on introduit dans le milieu de culture au moins un chromogène substrat d'une enzyme de la souche et au moins un hydrate de carbone à concentration élevée, afin d'obtenir après hydrolyse du chromogène une couleur différente de la couleur de base du chromophore.

Le milieu est avantageusement un milieu à base de peptone.

Par hydrate de carbone, on entend selon l'invention, l'ensemble des sucres naturels ou non, en particulier les oses, notamment pentoses ou hexoses, de préférence le glucose, à l'exception de tout composé hexosamin.

La concentration élevée en hydrate de carbone est de l'ordre de 10 à 30 g/l de milieu.

Par ailleurs, il a été également constaté que l'adjonction d'un phosphate à concentration élevée, lorsque l'une des enzymes est la phosphatase, permettait d'augmenter le nombre de couleurs dérivées susceptibles d'être obtenues par le procédé selon l'invention.

En conséquence, la présente invention concerne un procédé tel que défini précédemment, dans lequel le milieu de culture comprend une concentration élevée en phosphate, de préférence comprise entre 1 et 3g/l.

Les chromogènes sont notamment substrats des enzymes suivantes: β-galactosidase, β-glucuronidase, β-glucosidase, α-glucosidase, α-galactosidase, phosphatase, N-acétyl-β-glucosidase, N-acétyl-β-galactosidase, α-mannosidase, sulfatase, esterase, lipase et peptidase.

Le milieu comprend avantageusement au moins deux chromogènes, en particulier choisis parmi les composés d'une même famille chimique, de préférence parmi ceux qui libèrent par hydrolyse deux chromophores différents qui peuvent subir une réaction de couplage.

Par réaction de couplage, on entend toute interaction physico-chimique par laquelle la longueur d'onde dominante résultante est différente de la longueur d'onde dominante du mélange des deux chromophores pris isolément.

D'une manière préférentielle, les chromogènes sont de la famille des indoxyles, en particulier des dérivés indoxyle alkylés, halogénés ou dihalogénés.

Parmi les chromophores dérivés de l'indoxyle préférés, on trouve les dérivés jndoxyle, bromo-indoxyle, chloro-indoxyle, dichloro-indoxyle, chloro-bromo-indoxyle, tri-chloro-indoxyle et méthyl-indoxyle, en particulier les dérivés suivants: 6-chloro-indoxyle, 5-bromo-indoxyle, 3-bromo-indoxyle, 4,6-dichloro-indoxyle, 6,7-dichloro-indoxyle, 5-bromo-4-chloro-indoxyle, 5-bromo-6-chloro-indoxyle ou 4,6,7-trichloro-indoxyle.

Par microorganisme dont la présence ou l'absence est mise en évidence par le procédé selon l'invention, on entendra les levures, les champignons unicellulaires ou moisissures et les bactéries.

Le procédé selon l'invention est particulièrement adapté pour la mise en évidence de la présence ou l'absence des levures du genre Candida, en particulier Candida albicans et Candida tropicalis.

De même, parmi les bactéries dont la présence ou l'absence est susceptible d'être mise en évidence par le procédé selon l'invention, on trouve notamment les bactéries du genre Streptococcus, Klebsiella, Enterobacter, Escherichia, Citrobacter, Staphylococcus, Listeria, Clostridium ou Proteus.

Les exemples ci-après permettent d'illustrer le procédé selon l'invention sans toutefois chercher à en limiter la portée.

**Tableau I :**

| **Exemples de coloration dérivées** | | |
|---|---|---|
| **Chromogène** | **Milieu en g/l** | |
| | Agar 15 Na Cl5 Peptone 5 Extrait de viande 1 | Agar 15 Peptone 10 Glucose 20 |
| *5Bromo 4Chloro 3Indoxyl N acetyl glucosaminide 0,100* | *bleuâtre* | *vert clair*** |
| *5Bromo 6Chloro 3Indoxyl Nacetyl glucosaminide 0,100* | *rougeâtre* | *beige clair*** |
| *5Bromo 4Chloro 3Indoxyl phosphate 0,100* | *bleuâtre* | *vert clair*** |
| *5Bromo 6Chloro 3Indoxyl phosphate 0,100* | *rougeâtre* | *beige clair*** |
| *6Chloro 3 Indoxyl phosphate 0,200* | *rosâtre* | *blanchâtre*** |

| | | |
|---|---|---|
| ** couleur dérivée | | |

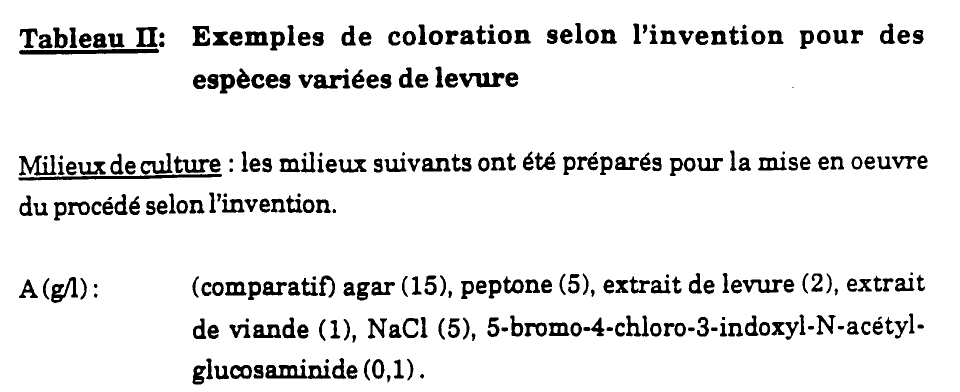

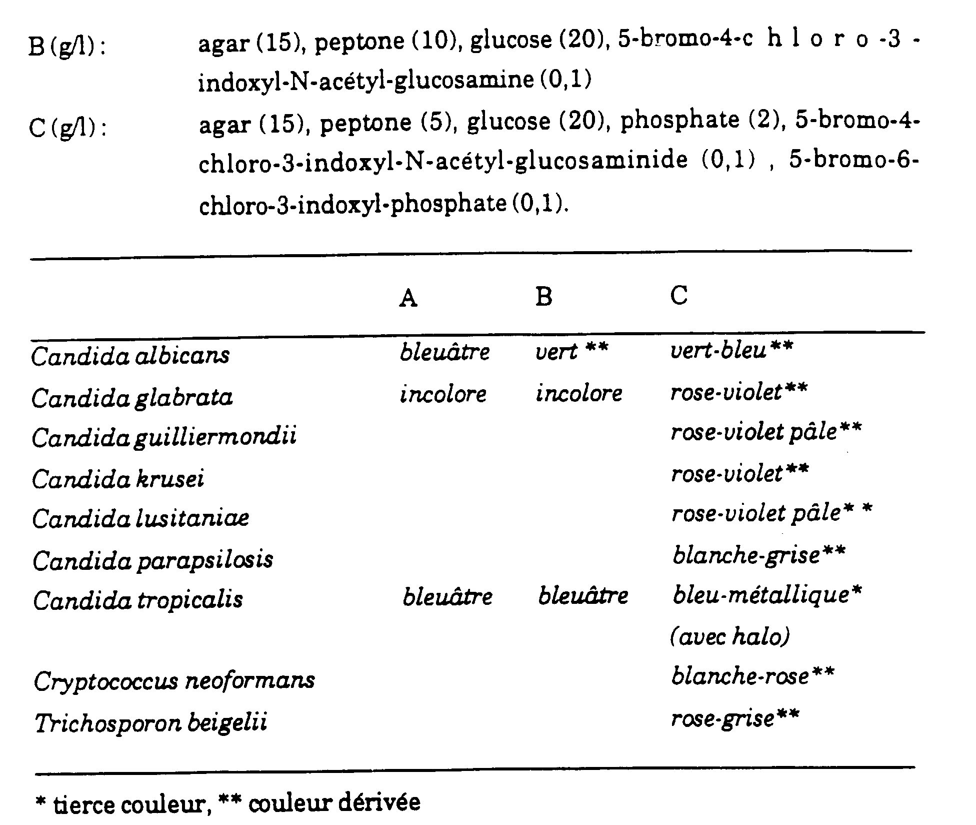

Les résultats ci-dessus montrent que l'adjonction de glucose et de phosphate permet d'élargir la gamme de couleurs disponibles pour un même milieu, permettant ici de distinguer sept espèces différentes, et en particulier de repérer Candida albicans sans ambiguité.

## Revendications

1. Procédé de mise en évidence de la présence ou de l'absence d'une souche de microorganisme particulière dans un milieu, caractérisé en ce qu'on introduit dans le milieu de culture au moins un chromogène substrat d'une enzyme de la souche et au moins un hydrate de carbone à concentration élevée, afin d'obtenir après hydrolyse du chromogène une couleur dérivée différente de la couleur de base du chromophore, étant entendu que l'hydrate de carbone n'est pas un composé hexosaminé.

2. Procédé selon la revendication 1, caractérisé en ce que le milieu est un milieu à base de peptone.

3. Procédé selon l'une des revendications 1 et 2, caractérisé en ce que la concentration en hydrate de carbone est de l'ordre de 10 à 30 g/l.

4. Procédé selon l'une des revendications 1 à 3, caractérisé en ce que le milieu comprend une concentration élevée en phosphate.

5. Procédé selon la revendication 4, caractérisé en ce que la concentration en phosphate est comprise entre 1 et 3 g/l.

6. Procédé selon l'une des revendications 1 à 5, caractérisé en ce que le milieu contient au moins deux chromogènes.

7. Procédé selon l'une des revendications 1 à 6, caractérisé en ce que les chromogènes sont des composés de la même famille chimique.

8. Procédé selon l'une des revendications 1 à 7, caractérisé en ce que les deux chromogènes libèrent par hydrolyse deux chromophores différents qui peuvent subir une réaction de couplage.

9. Procédé selon l'une des revendications 1 à 8, caractérisé en ce que les chromogènes sont de la famille des indoxyles, en particulier des dérivés indoxyle alkylés, halogénés ou dihalogénés.

10. Procédé selon l'une des revendications 1 à 9, caractérisé en ce que le chromophore est choisi parmi les dérivés indoxyle, bromo-indoxyle, chloro-indoxyle, dichloro-indoxyle, chloro-bromo-indoxyle, tri-chloro-indoxyle et méthyl-indoxyle, en particulier les dérivés suivants: 6-chloro-indoxyle, 5-bromo-indoxyle, 3-bromo-indoxyle, 4,6-dichloro-indoxyle, 6,7-dichloro-indoxyle, 5-bromo-4-chloro-indoxyle, 5-bromo-6-chlore-indoxyle ou 4,6,7-trichloro-indoxyle.

11. Procédé selon l'une des revendications 1 à 10, caractérisé en ce que les microorganismes sont des levures.

12. Procédé selon la revendication 10, caractérisé en ce que les levures sont du genre Candida.

13. Procédé selon l'une des revendications 1 à 10, caractérisé en ce que les microorganismes sont des bactéries.

14. Procédé selon la revendication 13, caractérisé en ce que les bactéries sont du genre Streptococcus, Klebsiella, Enterobacter, Escherichia, Citrobacter, Staphylococcus, Listeria, Clostridium ou Proteus.

## Claims

1. Method for demonstrating the presence or absence of a particular strain of microorganism in a medium, characterized in that at least one chromogen which is a substrate for an enzyme of the strain and at least one carbohydrate at high concentration are introduced into the culture medium, so as to obtain after hydrolysis of the chromogen a derived colour different from the basic colour of the chromophore, on the understanding that the carbohydrate is not a hexosamine compound.

2. Method according to Claim 1, characterized in that the medium is a peptone-based medium.

3. Method according to either of Claims 1 and 2, characterized in that the carbohydrate concentration is of the order of 10 to 30 g/l.

4. Method according to one of Claims 1 to 3, characterized in that the medium comprises a high phosphate concentration.

5. Method according to Claim 4, characterized in that the phosphate concentration is between 1 and 3 g/l.

6. Method according to one of Claims 1 to 5, characterized in that the medium contains at least two chromogens.

7. Method according to one of Claims 1 to 6, characterized in that the chromogens are compounds of the same chemical family.

8. Method according to one of Claims 1 to 7, characterized in that the two chromogens liberate on hydrolysis two different chromophores which can undergo a coupling reaction.

9. Method according to one of Claims 1 to 8, characterized in that the chromogens are of the indoxyl family, especially alkylated, halogenated or dihalogenated indoxyl derivatives.

10. Method according to one of Claims 1 to 9, characterized in that the chromophore is chosen from the indoxyl, bromoindoxyl, chloroindoxyl, dichloroindoxyl, chlorobromoindoxyl, trichloroindoxyl and methylindoxyl derivatives, and especially the following derivatives: 6-chloroindoxyl, 5-bromoindoxyl, 3-bromoindoxyl, 4,6-dichloroindoxyl, 6,7-dichloroindoxyl, 5-bromo-4-chloroindoxyl, 5-bromo-6-chloroindoxyl or 4,6,7-trichloroindoxyl.

11. Method according to one of Claims 1 to 10, characterized in that the microorganisms are yeasts.

12. Method according to Claim 10, characterized in that the yeasts are of the genus Candida.

13. Method according to one of Claims 1 to 10, characterized in that the microorganisms are bacteria.

14. Method according to Claim 13, characterized in that the bacteria are of the genus Streptococcus, Klebsiella, Enterobacter, Escherichia, Citrobacter, Staphylococcus, Listeria, Clostridium or Proteus.

## Patentansprüche

1. Verfahren zum Nachweis der Anwesenheit oder Abwesenheit eines speziellen Mikroorganismenstammes in einem Medium, dadurch gekennzeichnet, daß man in das Kulturmedium mindestens ein Farbbildner-Substrat eines Enzyms des Stammes sowie mindestens ein Kohlenhydrat in erhöhter Konzentration einführt, um nach der Hydrolyse des Farbbildners eine davon abgeleitete Farbe zu erhalten, die von der Grundfarbe des Farbbildners verschieden ist, wobei das Kohlenhydrat keine Hexosaminverbindung ist.

2. Verfahren nach Anspruch 1, dadurch gekennzeichnet, daß das Medium ein Medium auf Pepton-Basis ist.

3. Verfahren nach einem der Ansprüche 1 und 2, dadurch gekennzeichnet, daß die Kohlenwasserstoff-Konzentration in der Größenordnung von 10 bis 30 g/l liegt.

4. Verfahren nach einem der Ansprüche 1 bis 3, dadurch gekennzeichnet, daß das Medium eine erhöhte Phosphat-Konzentration aufweist.

5. Verfahren nach Anspruch 4, dadurch gekennzeichnet, daß die Phosphat-Konzentration zwischen 1 und 3 g/l liegt.

6. Verfahren nach einem der Ansprüche 1 bis 5, dadurch gekennzeichnet, daß das Medium mindestens zwei Farbbildner enthält.

7. Verfahren nach einem der Ansprüche 1 bis 6, dadurch gekennzeichnet, daß die Farbbildner Verbindungen aus der gleichen chemischen Familie sind.

8. Verfahren nach einem der Ansprüche 1 bis 7, dadurch gekennzeichnet, daß die beiden Farbbildner durch Hydrolyse zwei unterschiedliche Chromophore freisetzen, die eine Kupplungsreaktion eingehen können.

9. Verfahren nach einem der Ansprüche 1 bis 8, dadurch gekennzeichnet, daß die Farbbildner solche aus der Familie der Indoxyle, insbesondere der alkylierten, halogenierten oder dihalogenierten Indoxylderivate, sind.

10. Verfahren nach einem der Ansprüche 1 bis 9, dadurch gekennzeichnet, daß der Chromophor ausgewählt wird aus den Indoxyl-, Bromoindoxyl-, Chloroindoxyl-, Dichloroindoxyl-, Chlorobromoindoxyl-, Trichloroindoxyl- und Methylindoxyl-Derivate, insbesondere den 6-Chloro-indoxyl-, 5-Bromo-indoxyl-, 3-Bromo-indoxyl-, 4,6-Dichloro-indoxyl-, 6,7-Dichloro-indoxyl-, 5-Bromo-4-chloroindoxyl-, 5-Bromo-6-chloro-indoxyl- oder 4,6,7-Trichloro-indoxyl-Derivate.

11. Verfahren nach einem der Ansprüche 1 bis 10, dadurch gekennzeichnet, daß es sich bei den Mikroorganismen um Hefen handelt.

12. Verfahren nach Anspruch 11, dadurch gekennzeichnet, daß es sich bei den Hefen um solche des Genus Candida handelt.

13. Verfahren nach einem der Ansprüche 1 bis 10, dadurch gekennzeichnet, daß es sich bei den Mikroorganismen um Bakterien handelt.

14. Verfahren nach Anspruch 13, dadurch gekennzeichnet, daß es sich bei den Bakterien um solche der Genus Streptococcus, Klebsiella, Enterobacter, Escherichia, Citrobacter, Staphylococcus, Listeria, Clostridium oder Proteus handelt.
